# EUROPEAN PATENT SPECIFICATION

(11) **EP 0 492 556 B1**
(45) Date of publication and mention of the grant of the patent: **07.12.1994**
(21) Application number: 91122033.3
(22) Date of filing: 20.12.1991
(51) Int. Cl.: C07C 263/04

(54) **Continuous process for the preparation of isocyanates**
Kontinuierliches Verfahren zur Herstellung von Isocyanaten
Procédé de préparation en continu d'isocyanates

(30) Priority: 21.12.1990 IT 2247590
(43) Date of publication of application: 01.07.1992
(73) Proprietor: ENICHEM SYNTHESIS S.p.A., 90139 Palermo (IT)
(72) Inventor: Mizia, Franco, Dr., I-20097 San Donato Milanes, Milan (IT); Calderoni, Carlo, Dr., I-47100 Forli (IT)
(74) Representative: Barz, Peter, Dr.

(56) References cited:
- EP-A- 0 396 976
- US-A- 3 962 302
- PATENT ABSTRACTS OF JAPAN, vol. 10, no. 107 (C-341)(2164), 22 April 1986, & JP-A-60 237 058

## Description

The present invention relates to a process for the preparation of aliphatic or aromatic mono- and di-isocyanates by means of thermal decomposition of the corresponding urethanes.

Various processes for the preparation of isocyanates which are based on the decomposition reaction of the urethanes by operating in liquid phase and with a catalyst are known. In this decomposition an alcohol is formed which is a reaction co-product. In particular, JP-A-63/211257 describes a process for the preparation of isocyanates by thermal decomposition of the corresponding urethanes, carried out in a high boiling inert solvent and in the presence of metal catalysts such as vanadium and chromium. One of the reaction products is withdrawn from the decomposition reactor. Similarly, JP-A-63/211256 uses compounds of boron and metals of groups IA and IIA as catalysts. Also in this case one of the reaction products is removed from the decomposition reactor. WO-A-88/05430 describes a liquid phase process for the synthesis of aliphatic di-isocyanates which employs compounds of metals of groups VIA and VIIA as catalysts, at a temperature of 230°C and at reduced pressure. Both reaction products are removed from the decomposition reactor and recovered by means of two subsequent condensation steps. IT-A-20041 A/89 describes a process for the decomposition of urethanes which is carried out without a catalyst and in a boiling solvent. A low boiling solvent which forms an azeotropic mixture with the reaction co-product (the alcohol) is continuously fed to the decomposition reactor which is kept at 265°C. Both reaction products are continuously removed from the decomposition reactor and recovered in two subsequent condensation steps.

The basic problem in the decomposition of urethanes is the tendency of the reaction products, isocyanate and alcohol, to recombine and thereby reform the initial product. This fact, which causes difficulties in separating the reaction products and in recovering them in a sufficiently pure form, is a considerable drawback in the development of commercial scale processes. In catalytic decomposition processes for urethanes carried out in the liquid phase, there also is the problem of purifying the liquid from the catalytic residues.

The object of the present invention is to overcome the above drawbacks of the known processes.

It has now, surprisingly, been found that it is possible to decompose urethanes continuously and selectively by employing a liquid reaction mixture in which there are constantly limited quantities of unchanged urethane and isocyanate and by simultaneously eliminating the alcohol, the reaction co-product. It has also been found that it is possible to recover the isocyanate, continuously and in essentially pure form by partial evaporation of the liquid mixture, thus preventing the recombination of the reaction products.

Accordingly, the present invention provides a simple and convenient continuous process which can easily be scaled up to a commercial production and which affords isocyanates from the corresponding urethanes in high yields and with high selectivity.

More specifically, the present invention provides a continuous process for the production of mono- and di-isocyanates from the corresponding urethanes, according to the following reaction: 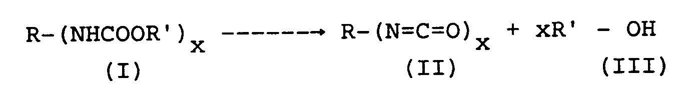
where R represents a group selected from linear or branched C₃-C₁₈ alkyl optionally having one or more ethylenic unsaturations; C₅-C₇ cycloalkyl; mononuclear or polynuclear aryl, with condensed or uncondensed rings; and aryl C₁-C₄ alkyl; said groups optionally having one or more substituents independently selected from C₁-C₄ alkyl, C₁-C₄ alkoxy, nitro, chloro, fluoro and bromo groups;
R' represents a C₁-C₂₀ alkyl group; and
x is 1 or 2;
said process comprising the steps of:
(a) continuously feeding a flow or urethane (I) to a decomposition reactor containing a liquid mixture which has an essentially constant volume and composition, does not contain any catalytic substances for the decomposition reaction, and comprises a high boiling, inert, organic solvent containing, per kg of liquid mixture, urethane (I) in a concentration of from 0.05 to 1.0 equivalent urethane functions and isocyanate (II) in a concentration of from 0.02 to 0.9 equivalent isocyanate functions; said reactor being kept at the boiling temperature of the solvent and at the transformation temperature of urethane (I) into isocyanate (II) and alcohol (III), the decomposition rate of the urethane (I) being comparable to the rate at which it is fed to the reactor, and the alcohol (III) being evaporated at a rate which essentially corresponds to its rate of formation;
(b) continuously withdrawing the liquid mixture from the reactor of step (a) at a rate which does not substantially change the volume of the mixture of step (a); feeding said mixture to a flash evaporator kept at a temperature which is lower than the decomposition temperature of the urethane (I), the isocyanate (II) being partially evaporated therein at a rate which is comparable to its rate of formation in the reactor of step (a); and recycling the residual liquid mixture from the flash evaporator to the reactor of step (a); and
(c) continuously withdrawing the vapor generated in step (b) and condensing and distilling said vapor in a distillation column to isolate isocyanate (II) as the head product of said column.

According to a preferred embodiment of the present process, R in the above formulae represents a group selected from aryl radicals deriving from aromatic monoamines, in particular aniline and aniline ortho-, meta- and/or para-substituted with alkyl groups such as methyl, ethyl, propyl, isopropyl and butyl; alkoxy groups and/or groups containing halogen atoms, in particular fluorine, chlorine and bromine; (cyclo)alkyl radicals deriving from (cyclo)aliphatic monoamines, in particular n-, i-, sec-, and tert-butyl amine, 2- and 3-methyl butyl amine, neopentyl amine, n-pentyl amine, n-hexyl amine, cyclohexyl amine, n-octyl amine and n-decyl amine; and alkyl radicals deriving from aliphatic diamines, in particular ethylene diamine, 1,3-propylene diamine, 1,4-butylene diamine, 1,5-pentylene diamine, 1,6-hexamethylene diamine and (3-aminomethyl-3,5,5-trimethylcyclohexyl)amine; and R' represents methyl.

Specific examples of urethanes (I) which can be subjected to the decomposition treatment according to the present invention are methyl N-phenyl urethane, methyl N-p-methoxyphenyl urethane, methyl N-p-chlorophenyl urethane, methyl N-3,5-dichlorophenyl urethane, methyl N-m-(trifluoromethyl) phenyl urethane, methyl N-cyclohexyl urethane, methyl N-isopropyl urethane, methyl N-butyl urethane, 1,3-bis(methoxycarbonylamino)-3,5,5-trimethyl cyclohexane and 1,6-dimethyl hexamethylene diurethane. Consequently, examples of isocyanates (II) are phenyl isocyanate, p-methoxyphenyl isocyanate, p-chlorophenyl isocyanate, 3,5-dichlorophenyl isocyanate, isopropyl isocyanate, cyclohexyl isocyanate, butyl isocyanate, m-(trifluoromethyl) isocyanate, isophorone diisocyanate and hexamethylene diisocyanate.

Preferably the high boiling inert organic solvent used in step (a) of the present process is an organic solvent whose boiling point is at least 40 to 50°C higher than that of the isocyanate (II), in such a way that it does not interfere with the decomposition reaction of the urethane (I). These high boiling organic solvents may be selected from aromatic hydrocarbons, such as alkylbenzenes containing from 9 to 14 carbon atoms in the linear of branched alkyl chain, methylnaphthalene, phenylnaphthalene, benzyl naphthalene and diphenyl; aliphatic hydrocarbons such as linear alkanes containing from 12 to 18 carbon atoms in the molecule; and ethers such as diphenyl ether, methylphenyl ether, cetylphenyl ether and p-nonylphenyl cetyl ether.

It is convenient in step (a) to operate under reflux conditions, preferably at a temperature of from 220 to 350°C, at atmospheric, reduced or superatmospheric pressure, and with a residence time in the reactor of from 1 to 20 minutes.

In step (b) it is convenient to operate at a temperature ranging from 150 to 200°C, partially evaporating the isocyanate, and consequently maintaining, in the residual liquid mixture, from 0.1 to 0.2 equivalent isocyanate functions and from 0.05 to 0.3 equivalent urethane functions, per kg of the mixture. The above residual liquid mixture is recycled to step (a), preferably with previous partial blowdown, in order to keep the content of impurities in the system at a constant or almost constant level. When this kind of blowdown is carried out, it is necesssary to also feed make-up solvent to step (a).

In step (c) the distillation is preferably carried out at a pressure which is sufficient to achieve, at a temperature of from 80 to 150°C, the condensation of the flow of isocyanate vapors at the head of the column. In the preferred method, the liquid removed from the bottom of the distillation column in step (c) is recycled to step (a).

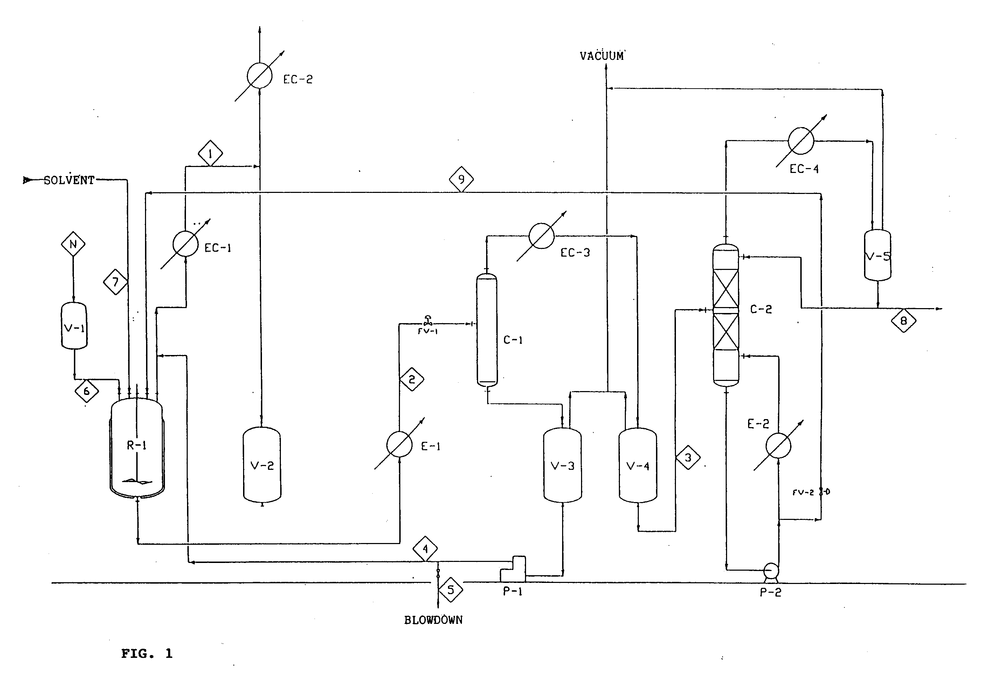

A preferred embodiment of the process of the present invention will now be described in some more detail with reference to Figure 1. In particular, in this figure, step (a) of the process is carried out in reactor R-1, which is kept at a temperature of from 220 to 350°C, under reflux of the reaction mixture, over a period ranging from 1 to 20 minutes. A sufficient amount of heat is supplied to result in an hourly amount (by weight) of vapor which is 1.5 to 2 times the amount by weight of liquid present in the reactor. These vapors are continuously condensed in EC-1 at a suitable temperature. A stream (6) composed of pure urethane and optionally a make-up amount of solvent (7) corresponding to the quantity discharged in (5) are continuously fed into R-1 which, under steady state conditions, contains from 0.3 to 0.5 equivalent isocyanate functions and from 0.2 to 0.5 equivalent urethane functions per kg of liquid. In addition, a recycle stream (4) from step (b) which, per kg of liquid, contains from 0.1 to 0.2 equivalent isocyanate functions and from 0.05 to 0.3 equivalent urethane functions in the reaction solvent is fed into reactor R-1 together with a stream (9) coming from step (c) which, per kg of liquid, contains from 0.02 to 0.9 equivalent isocyanate functions and from 0.05 to 1.0 equivalent urethane functions in the reaction solvent.

A stream of vapors (1) is continuously withdrawn from reactor R-1, and fractionated in EC-1, this stream being basically composed of alcohol reaction co-product and a reaction liquid stream (2) which, as specified above, contains from 0.2 to 0.5 equivalent isocyanate functions and from 0.1 to 0.3 equivalent urethane functions in the reaction solvent per kg of liquid.

Step (b) of the process is carried out in a flash chamber C-1, kept under non-reaction conditions, at a temperature ranging from 150 to 200°C and at reduced pressure. In particular, a liquid stream (2) having the above composition is continuously fed to C-1, after having been cooled in E-1, at a temperature ranging from 150 to 200°C. A stream of vapors (3) is withdrawn from C-1 in a weight ratio with respect to (2) of from 0.025/l to 0.2/l. This stream is condensed, either completely or partially, in EC-3 and contains, per kg of liquid, from 0.1 to 0.2 equivalent isocyanate functions and from 0.05 to 0.3 equivalent urethane functions in the solvent. In addition, a liquid stream (4) having the above composition is withdrawn and recycled by means of pump P-1 to step (a), after blowdown in (5). In the preferred method, the isocyanate, urethane and solvent will be recovered from this blowdown.

Step (c) of the process is carried out in the distillation column C-2, operating at a sufficient head pressure as to allow the condensation of the isocyanate within a temperature range of from 80 to 150°C. A liquid stream (3) having the above composition is continuously fed into C-2. A stream of vapors (8) is withdrawn from C-2, and is condensed in EC-4, in a weight ratio with respect to (3) of from 0.1/l to 0.5/l. This stream (8) is basically composed of pure isocyanate having the same molar content as that fed into (6). A liquid stream (9), having the above composition, is also withdrawn from C-2. This stream (9), as previously mentioned, is recycled to step (a).

The process of the present invention allows to produce isocyanate in highly selective manner, even without the use of a catalyst. In fact, the procedure described herein combines the advantages of a low conversion reaction by the flow of urethane and the continuous removal of the isocyanate from the system, affording an almost complete selectivity in the reaction main products. In addition, the separate removal of the two products of the decomposition reaction from the system carried out in two distinct but integrated phases results in a better yield of the product.

### EXAMPLE 1

In the present example, and in the examples which follow, the equipment illustrated in Figure 1 is used, where: R-1 is a stirring reactor for the thermal decomposition of the urethane (step (a)); C-1 is an adiabatic flash chamber (step (b)); C-2 is a continuous distillation column (step (c)); EC-1, EC-2, EC-3 and EC-4 are condensers; E-1 is an on-line heat exchanger; V-1 is the tank, with optional heating, for the urethane to be fed; V2, V4 and V5 are containers for collecting the condensates in the three steps of the process; V3 is a recycling receiver from step (b) to step (a) volumetrically operated by pump P-1. In particular, V3, V4 and V5 are equipped with a vacuum outlet. FV-1 and FV-2 are regulation valves for the flow of liquids from step (a) to step (b) and from step (c) to step (a), respectively. Condensers EC-1, EC-2, EC-3 and EC-4 are initially brought to the respective temperatures of 80, -10, 20 and 20°C. E-1 is brought to a temperature which is higher than the flash temperature but which is sufficient to block the reaction, normally to about 200°C.

R-1 in steady state condition contains 1.24 kg of liquid having the following composition: 93% by weight of inert solvent composed of a mixture of C₉-C₁₄-alkylbenzenes (on the average C₁₀-alkylbenzene, hereinafter referred to as DDB), 2.2 to 3.4% by weight of cyclohexyl isocyanate (CHI), 3.1 to 1.5% by weight of methyl N-cyclohexyl urethane (CHU) and 2.2% by weight of other products.

R-1 operates at the boiling point of the liquid at 265°C with such a supply of heat as to obtain a stream of condensed vapors form EC-1 equal to 4.3 kg/hour. Moreover, the average operating residence time of R-1 is 12.0 minutes (calculated as the ratio of the volumetric feeding capacity and the weight of the stationary liquid in the reactor). C-1 operates under adiabatic conditions by feeding a liquid kept at 200°C into E-1, with a contact time of about 2 seconds. Moreover, C-1 operates at a pressure which is sufficient to obtain a flow of condensed vapors into EC-3 in a weight ratio with respect to the feed of 0.03/l; said pressure is normally about 13,3 kPa (100 mm Hg). C-2 operates at a pressure, measured at the head, which is sufficient to cause the condensation of CHI at about 85°C, normally at about 6,6 kPa (50 mm Hg), and with a supply of heat as to obtain a flow of condensed vapors in EC-4 in a weight ratio with respect to the feed at C2 of 0.5/l, with a reflux ratio of 1.5/l.

Under these conditions, a stream (6) of 0.1 kg/hour of 99.6% by weight of CHU (liquid at 75°C) is fed into R-1, which under the conditions applied to the system undergoes complete conversion. In addition, a stream (7) of 0.038 kg/hour of fresh DDB is continuously fed into R-1. A recycled stream (4) composed of 2.3% by weight of CHI, 1.36% by weight of CHU, 2.27% by weight of other products and 94.0% by weight of DDB (after a discharge of 0.04 kg/hour in (5)), is reintroduced into reactor R-1 at a flow rate of 5.99 kg/hour. Finally, a stream (9) composed of 0.44% by weight of CHI, 12.6% by weight of CHU and 87% by weight of DDB is fed from step (c) to reactor R-1 in quantities of 0.09 kg/hour.

A stream of vapors (1) (fractionated in EC-1), of which 99.4% by weight are composed of methanol and the remainder is CHU, is withdrawn continuously from reactor R-1 in quantities of 0.019 kg/hour. A liquid reaction stream (2) composed of 3.4% by weight of CHI, 1.5% by weight of CHU, 2.2% by weight of other products and 98.2% by weight of DDB is also continuously withdrawn from reactor R-1 with a flow rate of 6.2 kg/hour.

Stream (2) is continuously fed to C1 from which a liquid stream (4) having the above composition is withdrawn with a flow rate of 6.04 kg/hour. A stream of vapors (3) composed of 45.1% by weight of CHI, 6.98% by weight of CHU and 47.8% by weight of DDB is also withdrawn with an hourly flow rate of 0.16 kg.

This latter stream (3) is continuously fed to C2, from which a liquid stream (9) having the above composition is withdrawn together with a stream of vapors (8) which are condensed and withdrawn with a flow rate of 0.0739 kg/hour, comprising the main reaction product with a 99% by weight purity.

From the above data it can be calculated that, under steady state conditions:
- the conversion of CHU, per run, is equal to 51.4%;
- the selectivity with respect to CHI is equal to 94.6%;
- the productivity is equal to 59.6 g of CHI per litre of reactor and per hour.

### EXAMPLE 2

The same procedure as in example 1 is used with the equipment of Figure 1. In particular, a stream (6) of FMU (methyl N-phenylurethane) is fed to R-1 at 0.114 kg/hour which, under the conditions applied, is completely converted. In addition, a stream of fresh DDB (7) is fed into R-1 at 0.0479 kg/hour. A recycled stream (4) composed of 1.85% by weight of FI (phenyl isocyanate), 4.21% by weight of FMU, 2.88% by weight of other products and 91% by weight of DDB is also fed from step (b) (after a discharge of 0.05 kg/hour in (5)) to R-1 with a flow rate of 5.91 kg/hour. Finally, a stream (9) composed of 1.76% by weight of FI, 10.5% by weight of FMU and 87.3% by weight of DDB is fed from step (c) to reactor R-1 at a rate of 0.085 kg/hour.

A stream of vapors (1) (fractionated in EC-1), of which 99.5% are methanol and the remainder is FMU, is continuously withdrawn from reactor R-1 at a rate of 0.0236 kg/hour. A liquid reaction stream (2) composed of 3.2% by weight of FI, 4.24% by weight of FMU, 2.81% by weight of other products and 89.7% by weight of DDB is continuously withdrawn form reactor R-1 with a flow rate of 6.13 kg/hour.

Stream (2) is continuously fed to C-1, from which a liquid stream (4) having the above composition is withdrawn with a flow rate of 5.96 kg/hour. A stream of vapors (3) composed of 50.6% by weight of FI, 5.3% by weight of FMU and 43.6% by weight of DDB is also withdrawn with a flow rate of 0.17 kg/hour and condensed.

This latter stream is continuously fed to C-2, from which a liquid stream (9) having the above composition is withdrawn together with a stream of vapors (8) which are condensed and withdrawn with a flow rate of 0.0845 kg/hour and comprise the main reaction product with a 99% purity by weight.

From the above data it can be calculated that under steady state conditions:
- the conversion of FMU, per run, is equal to 30.1%,
- the selectivity with respect to FI is equal to 96.7%, and
- the productivity is equal to 68.14 g of FI per litre of reactor and per hour.

### EXAMPLE 3

The equipment of Figure 1 is used under the conditions described in example 1, but keeping a temperature of 280°C and a residual pressure of 25,3 kPa (190 mm Hg) in R-1. In particular, a stream (6) of 0.156 kg/hour of NDU (1,6-dimethyl hexamethylenediurethane) is fed to R-1 and, under the conditions applied, is completely converted. In addition, a recycled stream (4) is fed into R-1 from step (b) with a flow rate of 5.07 kg/hour and with the following composition: 0.53% by weight of HDI (1,6-hexamethylene diisocyanate), 2.09% by weight of HMI (hexamethylene monoisocyanate monourethane), 1.01% by weight of HDU and 96.4% by weight of solvent (diphenyl alkylate). Finally, a stream (9) composed of 11.45% by weight of HMI, 0.36% by weight of HDU and 88.2% by weight of solvent is fed from step (c) to reactor R-1 in quantities of 0.961 kg/hour.

A stream of vapors (1) (fractionated in EC-1) of which 99.5% consist of methanol and the remainder is HDU and small fractions of solvent is continuously withdrawn from reactor R-1 at a rate of 0.043 kg/hour. A liquid reaction stream (2) composed of 2.27% by weight of HDI, 3.51% by weight of HMI, 0.89% by weight of HDU and 93.3% by weight of solvent is continuously removed from reactor R-1 with a flow rate of 6.14 kg/hour.

Stream (2) is continuously fed to C-1, from which a liquid stream (4) having the above composition is removed with a flow rate of 5.07 kg/hour. A stream of vapors (3) composed of 10.5% by weight of HDI, 10.2% by weight of HMI, 0.32% by weight of HDU and 78.9% by weight of solvent is also removed and condensed at a rate of 1.075 kg/hour.

The latter stream is continuously fed to C-2, from which a liquid stream (9) of the above composition is withdrawn together with a stream of vapors (8) which are condensed and withdrawn at a rate of 0.113 kg/hour, said vapors comprising the main reaction product with a 99% purity by weight.

From the above data it can be calculated that under steady state conditions:
- the conversion of HDU, per run, is equal to 74%,
- the selectivity with respect to HMI is equal to 71.6%,
- the selectivity with respect to HDI is equal to 28.4% and
- the productivity is equal to 91 g of HDI per litre of reactor and per hour.

## Claims

1. Continuous process for the preparation of organic mono- and di-isocyanates of general formula (II) from the corresponding urethane of general formula (I) and under additional formation of alcohol of general formula (III): 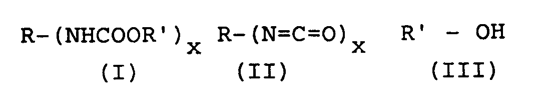 where R represents a group selected from linear or branched C₃-C₁₈ alkyl optionally having one or more ethylenic unsaturations; C₅-C₇ cycloalkyl; mononuclear or polynuclear aryl, with condensed or uncondensed rings; and aryl C₁-C₄ alkyl; said groups optionally having one or more substituents independently selected from C₁-C₄ alkyl, C₁-C₄ alkoxy, nitro, chloro, fluoro and bromo groups;
R' represents a C₁-C₂₀ alkyl group; and
x is 1 or 2;
said process comprising the steps of:
(a) continuously feeding a flow or urethane (I) to a decomposition reactor containing a liquid mixture which has an essentially constant volume and composition, does not contain any catalytic substances for the decomposition reaction, and comprises a high boiling, inert, organic solvent containing, per kg of liquid mixture, urethane (I) in a concentration of from 0.05 to 1.0 equivalent urethane functions and isocyanate (II) in a concentration of from 0.02 to 0.9 equivalent isocyanate functions; said reactor being kept at the boiling temperature of the solvent and at the transformation temperature of urethane (I) into isocyanate (II) and alcohol (III), the decomposition rate of the urethane (I) corresponding essentially to the rate at which it is fed to the reactor, and the alcohol (III) being evaporated at a rate which essentially corresponds to its rate of formation;
(b) continuously withdrawing the liquid mixture from the reactor of step (a) at a rate which does not substantially change the volume of the mixture of step (a); feeding said mixture to a flash evaporator kept at a temperature which is lower than the decomposition temperature of the urethane (I), the isocyanate (II) being partially evaporated therein at a rate which essentially corresponds to its rate of formation in the reactor of step (a); and recycling the residual liquid mixture from the flash evaporator to the reactor of step (a); and
(c) continuously withdrawing the vapor generated in step (b) and condensing and distilling said vapor in a distillation column to isolate isocyanate (II) as the head product of said column.

2. Process according to claim 1, wherein R represents a group selected from aryl radicals deriving from aromatic monoamines, particularly aniline and aniline ortho-, meta-, and/or para-substituted with (halo)alkyl groups and/or alkoxy groups and/or halogen atoms; (cyclo)alkyl radicals deriving from (cyclo)aliphatic monoamines, particularly n-, i-, sec- and tert-butyl amino, 2- and 3-methyl butyl amine, neopentyl amine, n-pentyl amine, n-hexyl amine, cyclohexyl amine, n-octyl amine and n-decyl amine; and alkyl radicals deriving from aliphatic diamines, particularly ethylene dianine, 1,3-propylene diamine, 1,4-butylene diamine, 1,5-pentylene diamine, 1,6-hexamethylene diamine and (3-aminomethyl-3,5,5,-trimethylcyclohexyl)amine.

3. Process according to claim 2, wherein the (halo)alkyl and alkoxy substituents of aniline are selected from methyl, ethyl, propyl, isopropyl, butyl, the corresponding alkoxy groups, and alkyl groups containing one or more atoms selected from fluorine, chlorine and bromine.

4. Process according to any one of claims 1 to 3, wherein R' represents methyl.

5. Process according to claim 1, wherein urethane (I) is selected from methyl N-phenyl urethane, methyl N-p-methoxyphenyl urethane, methyl N-p-chlorophenyl urethane, methyl N-m-(trifluoromethyl)phenyl urethane, methyl N-cyclohexyl urethane, methyl N-butyl urethane, 1,3-bis-(methoxycarbonylamino)-3,5,5,-trimethylcyclohexane and 1,6-dimethyl hexamethylene diurethane.

6. Process according to any one of claims 1 to 5, wherein the solvent employed in step (a) has a boiling point which is at least 40°C higher than that of the isocyanate (II) and is selected from aromatic hydrocarbons, particularly alkylbenzenes containing from 9 to 14 carbon atoms in the linear or branched alkyl chain, methylnaphthalene, phenylnaphthalene, benzylnaphthalene and diphenyl; aliphatic hydrocarbons, particularly linear alkanes containing from 12 to 18 carbon atoms in the molecule; and ethers, particularly diphenyl ether, methylphenyl ether, cetylphenyl ether and p-nonylphenyl cetyl ether.

7. Process according to any one of claims 1 to 6, wherein in step (a) the operating temperature ranges from 220 to 350°C and the residence time is from 1 to 20 minutes.

8. Process according to any one of claims 1 to 7, wherein in step (b) the operating temperature ranges form 150 to 200°C and the isocyanate is partially evaporated, constantly maintaining, in the residual liquid mixture, a concentration of from 0.1 to 0.2 equivalent isocyanate functions and from 0.05 to 0.3 equivalent urethane functions per kg of mixture.

9. Process according to any one of claims 1 to 8, wherein the liquid mixture recycled from step (b) to step (a) is subjected to partial blowdown so as to keep the content of impurities in the system at a constant or almost constant level, and an amount of make-up solvent essentially equal to the amount discharged is also fed to step (a).

10. Process according to any one of claims 1 to 9, wherein in step (c) the distillation is carried out at a temperature of from 80 to 150°C and at a pressure which is sufficient to cause the condensation of the isocyanate vapors at the head of the column, the residual liquid mixture of the distillation being recycled to step (a).

## Patentansprüche

1. Kontinuierliches Verfahren zur Herstellung von organischen Mono- und Diisocyanaten der allgemeinen Formel (II) aus dem entsprechenden Urethan der allgemeinen Formel (I) und unter zusätzlicher Bildung eines Alkohols der allgemeinen Formel (III): 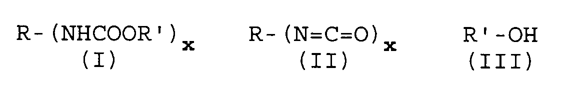 worin R eine Gruppe, ausgewählt aus linearem oder verzweigtem C₃-C₁₈ Alkyl, welches gegebenenfalls ein oder mehrere ethylenische Ungesättigtheiten aufweist; C₅-C₇ Cycloalkyl; ein- oder mehrkernigem Aryl mit kondensierten oder nicht-kondensierten Ringen; und Aryl-C₁-C₄-alkyl bedeutet; wobei diese Gruppen gegebenenfalls einen oder mehrere Substituenten aufweisen, die unabhängig ausgewählt sind aus C₁-C₄ Alkyl-, C₁-C₄ Alkoxy-, Nitro-, Chlor-, Fluor- und Brom-Gruppen;
R' eine C₁-C₂₀ Alkylgruppe darstellt; und
x 1 oder 2 ist;
welches Verfahren die Schritte umfaßt:
(a) kontinuierliches Einspeisen eines Stromes von Urethan (I) in einen Zersetzungsreaktor, der eine flüssige Mischung von im wesentlichen konstantem Volumen und Zusammensetzung enthält, keine katalytische Substanzen für die Zersetzungsreaktion enthält und ein hochsiedendes, inertes, organisches Lösungsmittel umfaßt, enthaltend pro kg der flüssigen Mischung Urethan (I) in einer Konzentration von 0,05 bis 1,0 Äquivalent Urethan-Funktionen und Isocyanat (II) in einer Konzentration von 0,02 bis 0,9 Äquivalent Isocyanat-Funktionen; wobei der Reaktor bei der Siedetemperatur des Lösungsmittels und bei der Transformationstemperatur des Urethans (I) in das Isocyanat (II) und den Alkohol (III) gehalten wird, die Zersetzungsrate des Urethans (I) im wesentlichen der Rate entspricht, mit der es in den Reaktor eingespeist wird, und der Alkohol (III) mit einer Rate verdampft wird, die im wesentlichen seiner Bildungsrate entspricht;
(b) kontinuierliches Abziehen der flüssigen Mischung aus dem Reaktor von Schritt (a) mit einer Rate, die im wesentlichen nicht das Volumen der Mischung von Schritt (a) ändert; Einspeisen der Mischung in einen Flash-Verdampfer, der bei einer niedrigeren Temperatur als der Zersetzungstemperatur des Urethans (I) gehalten wird, wobei das Isocyanat (II) darin mit einer Rate, die im wesentlichen seiner Bildungsrate in dem Reaktor von Schritt (a) entspricht, teilweise verdampft wird; und Recyclieren der restlichen flüssigen Mischung aus dem Flash-Verdampfer in den Reaktor von Schritt (a); und
(c) kontinuierliches Abziehen des in Schritt (b) entwickelten Dampfes und Kondensieren und Destillieren des Dampfes in einer Destillationskolonne, um Isocyanat (II) als Kopfprodukt der Kolonne zu isolieren.

2. Verfahren nach Anspruch 1, worin R eine Gruppe bedeutet, ausgewählt aus Arylresten, die sich von aromatischen Monoaminen, insbesondere Anilin und Anilin, welches mit (Halogen)alkylgruppen und/oder Alkoxygruppen und/oder Halogenatomen ortho-, meta- und/oder para-substituiert ist, ableiten; (Cyclo)alkylreste, die sich von (cyclo)aliphatischen Monoaminen, insbesondere n-, i-, sek- und tert-Butylamin, 2- und 3-Methylbutylamin, Neopentylamin, n-Pentylamin, n-Hexylamin, Cyclohexylamin, n-Octylamin und n-Decylamin, ableiten; und Alkylresten, die sich von aliphatischen Diaminen, insbesondere Ethylendiamin, 1,3-Propylendiamin, 1,4-Butylendiamin, 1,5-Pentylendiamin, 1,6-Hexamethylendiamin und (3-Aminomethyl-3,5,5-trimethylcyclohexyl)amin, ableiten.

3. Verfahren nach Anspruch 2, worin die (Halogen)alkyl- und Alkoxysubstituenten des Anilins ausgewählt sind aus Methyl, Ethyl, Propyl, Isopropyl, Butyl, den entsprechenden Alkoxygruppen und Alkylgruppen, die ein oder mehrere Atome, ausgewählt aus Fluor, Chlor und Brom, enthalten.

4. Verfahren nach irgendeinem der Ansprüche 1 bis 3, worin R' Methyl bedeutet.

5. Verfahren nach Anspruch 1, worin das Urethan (I) ausgewählt ist aus Methyl-N-phenylurethan, Methyl-N-p-methoxyphenylurethan, Methyl-N-p-chlorphenylurethan, Methyl-N-m-(trifluormethyl)phenylurethan, Methyl-N-cyclohexylurethan, Methyl-N-butylurethan, 1,3-Bis(methoxycarbonylamino)-3,5,5-trimethylcyclohexan und 1,6-Dimethylhexamethylendiurethan.

6. Verfahren nach irgendeinem der Ansprüche 1 bis 5, worin das in Schritt (a) angewandte Lösungsmittel einen Siedepunkt hat, der mindestens 40°C höher ist als der des Isocyanats (II), und ausgewählt ist aus aromatischen Kohlenwasserstoffen, insbesondere Alkylbenzolen mit 9 bis 14 Kohlenstoffatomen in der linearen oder verzweigten Alkylkette, Methylnaphthalin, Phenylnaphthalin, Benzylnaphthalin und Diphenyl; aliphatischen Kohlenwasserstoffen, insbesondere linearen Alkanen mit 12 bis 18 Kohlenstoffatomen im Molekül; und Ethern, insbesondere Diphenylether, Methylphenylether, Cetylphenylether und p-Nonylphenylcetylether.

7. Verfahren nach irgendeinem der Ansprüche 1 bis 6, worin in Schritt (a) die Betriebstemperatur im Bereich von 220 bis 350°C liegt und die Verweilzeit 1 bis 20 Minuten beträgt.

8. Verfahren nach irgendeinem der Ansprüche 1 bis 7, worin in Schritt (b) die Betriebstemperatur im Bereich von 150 bis 200°C liegt und das Isocyanat teilweise verdampft wird, wobei man in der restlichen flüssigen Mischung eine Konzentration von 0,1 bis 0,2 Äquivalent Isocyanat-Funktionen und 0,05 bis 0,3 Äquivalent Urethan-Funktionen pro kg Mischung konstant aufrechterhält.

9. Verfahren nach irgendeinem der Ansprüche 1 bis 8, worin die flüssige Mischung, welche von Schritt (b) zu Schritt (a) recycliert wird, teilweise niedergeschlagen wird, um den Gehalt an Verunreinigungen in dem System auf einem konstanten oder fast konstanten Niveau zu halten, und eine Menge an Ergänzungslösungsmittel, die im wesentlichen gleich ist der abgezogenen Menge, ebenfalls in Schritt (a) zugeführt wird.

10. Verfahren nach irgendeinem der Ansprüche 1 bis 9, worin in Schritt (c) die Destillation bei einer Temperatur von 80 bis 150°C und einem Druck durchgeführt wird, der ausreicht, die Kondensation der Isocyanatdämpfe am Kolonnenkopf zu bewirken, wobei die restliche flüssige Mischung der Destillation zu Schritt (a) recycliert wird.

## Revendications

1. Procédé continu de préparation de mono- et di-isocyanates représenté par la formule générale (II) à partir des uréthanes correspondants de formule générale (I) et avec formation d'alcool de formule générale (III): 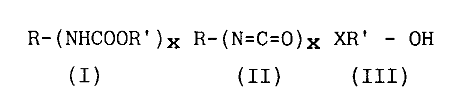 dans laquelle:
R représente un groupe sélectionné parmi les radicaux alkyle linéaires ou ramifiés en C₃ à C₁₈ comportant éventuellement une ou plusieurs insaturations éthyléniques; cycloalkyle en C₅ à C₇; aryle mononucléaire ou polynucléaire; avec des cycles condensés ou non condensés; et aryl-alkyle en C₁ à C₄; lesdits groupes comportant éventuellement un ou plusieurs substituants qui sont indépendamment sélectionnés parmi les radicaux alkyles en C₁ à C₄, alcoxy en C₁ à C₄, groupes nitro, chloro, fluoro et bromo;
R' représente un radical alkyle en C₁ à C₂₀; et
X est égal à 1 ou 2;
ledit procédé comprenant les étapes de:
a) chargement en continu d'un flux d'uréthane (I) dans un réacteur de décomposition contenant un mélange liquide qui présente une composition et un volume pratiquement constants, ne contient aucune substance catalytique pour la réaction de décomposition, et comprend un solvant organique, inerte, à haut point d'ébullition, contenant, par kg de mélange liquide, de l'uréthane (I) à une concentration de 0,05 à 1,0 équivalent de fonctions uréthane et de l'isocyanate (II) à une concentration de 0,02 à 0,9 équivalent de fonctions isocyanate; ledit réacteur étant maintenu à la température d'ébullition du solvant et à la température de transformation de l'uréthane (I) en isocyanate (II) et en alcool (III), la vitesse de decomposition de l'uréthane (I) étant comparable à la vitesse à laquelle il est chargé dans le réacteur, et l'alcool (III) étant évaporé à une vitesse qui correspond sensiblement à sa vitesse de formation;
b) élimination en continu du mélange liquide à partir du réacteur de l'étape (a) à une vitesse qui ne modifie pas sensiblement le volume du mélange de l'étape (a); chargement dudit mélange dans un évaporateur flash maintenu à une température qui est inférieure à la température de décomposition de l'uréthane (I), l'isocyanate (II) étant partiellement évaporé à une vitesse qui est comparable à sa vitesse de formation dans le réacteur de l'étape (a); et recyclage du mélange liquide résiduel à partir de l'évaporateur flash vers le réacteur de l'étape (a); et
c) élimination en continu de la vapeur générée à l'étape (b) et condensation puis distillation de ladite vapeur dans une colonne de distillation pour isoler l'isocyanate (II) en tant que produit de tête de ladite colonne.

2. Procédé selon la revendication 1, dans lequel R représente un groupe sélectionné parmi les radicaux aryles dérivant de monoamines aromatiques, en particulier aniline et aniline ortho-, méta- et/ou para-substituées par des radicaux (halo)alkyles et/ou alcoxy et/ou des atomes d'halogène; radicaux (cyclo)alkyles dérivant de monoamines (cyclo)aliphatiques, en particulier n-, i-, sec- et tertbutylamine, 2- et 3-méthylbutylamine, néopentylamine, n-pentylamine, n-hexylamine, cyclohexyl-amine, n-octylamine et n-décylamine; et radicaux alkyles dérivant de diamines aliphatiques, en particulier éthylènediamine, 1,3-propylènediamine, 1,4-butylènediamine, 1,5-pentylènediamine, 1,6-hexaméthylènediamine et (3-aminométhyl-3,5,5-triméthylcyclohexyl) amine.

3. Procédé selon la revendication 2, dans lequel les substituants (halo)alkyle et alcoxy de l'aniline sont sélectionnés parmi les groupes méthyle, éthyle, propyle, isopropyle, butyle, les groupes alcoxy correspondants, et les groupes alkyles contenant un ou plusieurs atomes sélectionnés parmi fluor, chlore et brome.

4. Procédé selon l'une quelconque des revendications 1 à 3, dans lequel R' représente un radical méthyle.

5. Procédé selon la revendication 1, dans lequel l'uréthane (I) est sélectionnée parmi: méthyl-N-phényl-uréthane, méthyl-N-p-méthoxyphényluréthane, méthyl-N-p-chlorophényluréthane, méthyl-N-m-(trifluorométhyl)phényl-uréthane, méthyl-N-cyclohexyluréthane, méthyl-N-butyluréthane, 1,3-bis-(méthoxycarbonylamino)-3,5,5-triméthylcyclohexane et 1,6-diméthyl-hexaméthylène diuréthane.

6. Procédé selon l'une quelconque des revendications 1 à 5, dans lequel le solvant utilisé dans l'étape (a) présente un point d'ébullition qui est au moins 40°C supérieur à celui de l'isocyanate (II) et est sélectionné parmi les hydrocarbures aromatiques, particulièrement les alkylbenzènes contenant de 9 à 14 atomes de carbone au niveau de la chaîne alkyle linéaire ou ramifiée, méthylnapthalène, phénylnaphtalène, benzylnaphtalène et diphényle; hydrocarbures aliphatiques, plus particulièrement les alcanes linéaires contenant de 12 à 18 atomes de carbone dans la molécule; et les éthers, plus particulièrement diphényléther, méthylphényléther, cétylphényléther et p-nonylphénylcétyléther.

7. Procédé selon l'une quelconque des revendications 1 à 6, dans lequel dans l'étape (a), la température opératoire est de 220 à 350°C et le temps de séjour est de 1 à 20 minutes.

8. Procédé selon l'une quelconque des revendications 1 à 7, dans lequel dans l'étape (b), la température opératoire est de 150 à 200°C et l'isocyanate est partiellement évaporé, en maintenant constamment dans le mélange liquide résiduel une concentration de 0,1 à 0,2 équivalent de fonctions isocyanate et de 0,05 à 0,3 équivalent de fonctions uréthane par kg de mélange.

9. Procédé selon l'une quelconque des revendications 1 à 8, dans lequel le mélange liquide recyclé de l'étape (b) vers l'étape (a) est soumis à une élimination partielle de façon à maintenir la teneur des impuretés dans le système à un taux constant ou pratiquement constant, et une quantité de solvant d'appoint essentiellement égale à la quantité déchargée est également chargée dans l'étape (a).

10. Procédé selon l'une quelconque des revendications 1 à 9, dans lequel dans l'étape (c), la distillation est mise en oeuvre à une température de 80 à 150°C et à une pression qui est suffisante pour provoquer la condensation des vapeurs d'isocyanate au sommet de la colonne, le mélange liquide résiduel de la distillation étant recyclé dans l'étape (a).
